# EUROPEAN PATENT APPLICATION

(11) **EP 0 655 229 A1**
(43) Date of publication of application: **31.05.1995**
(21) Application number: 94500193.1
(22) Date of filing: 23.11.1994
(51) Int. Cl.: A61F 2/30, A61F 2/36

(54) **Straight non-hardened stem for total hip prostheses**

(30) Priority: 25.11.1993 ES 9302483
(71) Applicant: INDUSTRIAS QUIRURGICAS DE LEVANTE, S.A., 46988 Paterna - Valencia (ES)
(72) Inventor: Cogollos, Bernardo, E-46988 Paterna, Valencia (ES); Bisquert Mahiques, Jose Luis, 46988 Paterna - Valencia (ES)
(74) Representative: Urizar Anasagasti, José Antonio

(57) **Abstract**

A stem for total hip prosthesis of straight axis constructed with a material of exceptional bio-compatibility and high resistance against fatigue such as Ti-6Al-4V alloy and introduced in a modular system, which due to its advanced design presents a geometrical shape of excelent stability in the reconstruction of the articulation of the hip. This stem has a coating of inlayed poropatite (porous coating + hydroxyapatite) and with support collar at the calcareous. Thus making possible the surgical technics of the same to be simple and precise, using instrumentation corresponding to the hip modular system.

## Description

The present invention relates to a new prototype of a straight non-hardened stem for total hip prosthesis with inlayed coating and support collar at calcareous.

A good hip prosthesis not only has to comply a series of basic principles, but it has to have a simple and accurate technic. In this manner, technical errors which limit the life of the prosthesis are avoided.

Comparing the modular prosthesis object of the present invention with those existing at present one comes to:

### Mechanical and physical properties:

The mechanical and physical benefits in an implant have great importance, for example, in table I it can be observed, from the different employed materials in surgical implants, that the highest levels of hardness correspond to titanium alloys (Ti-6Al-4V).

**TABLA I**

| MATERIAL | ROCKWELL HARDNESS | Tension Rupture Nw/mm² | Elastic limit Nw/mm² |
|---|---|---|---|
| -AISI 316 LVM (cold-rolled) | 30 | 965 | 792 |
| -Cr-Co-MO (casted) | 29 | 689 | 517 |

| -Ti-6Al-4V | | | |
|---|---|---|---|
| (forged) | 35 | 965 | 896 |
| (forged and with thermal treatment) | 39 | 1102 | 1033 |
| (casted) | 35 | 896 | 827 |

**TABLE II**

| Material | Density | Elastic Module (Nw/mm²) | Resistance (10⁷ Cycle) Nw/mm² |
|---|---|---|---|
| -AISI 316 LVM cold -rolled | 8.0 | 190.000÷210.000 | 310 - 448 |
| -Cr-Co-Mo casted | 8.3 | 250.000÷270.000 | 241 - 310 |
| -Ti-6Al-4V | 4.4 | 110.000÷124.000 | |
| Forged | | | 517 - 551 |
| Forged + T.T. | | | 620 - 689 |
| Casted | | | 241 - 345 |

The higer values for the tension of rupture and elastic limits in the Ti alloy mean that with iqual cross-sections the elastic behaviour of Ti alloy is produced in a lager field of tensions, that is, the values of tensions which produce in Co-Cr-Mo and AISI 316 LVM permanent deformations, do not produce the same in Ti-6Al-4V and the latter will continue an elastic behaviour, that is, it recovers theoriginal dimensions thereof.

The great difference between the bone elasticity modules and the AISI 316 LVM y Cr-Co-Mo, involves that for a given pressure, the bone will suffer different deformations than that of the prosthesis involving the danger of loosening of the latter inside the bone, which could give way to resorption of the bone or necrosis. The difference between the bone and the Ti-6Al-4V is approximately half by reducing considerably the mentioned risk, in addition, this proximity between modules of elasticity implies that the system bone-prosthesis can flex more degrees than other alloys for a determined tension and therefor reduces the tension transferred to the bone.

Resistance to the fatigue is an important characteristic of the raw material which is used in an implant, especially if it will be subjected to cyclic charges.
it can be observed from table II that the alloy of Ti has a resistance to the fatigue of almost twice as much.

The density is a physical property which isto be taken into account. Observing table II, it can be seen that the alloy of Ti has a density value of half of therest of the implantable material, which provides for prosthesis considerably lighter to the benefit of implant carrying patients.

The surgical implants, must have a good resistance against corrosion which may be generated inside the fluids of the organism. From experiences and studies performed, the outstanding behaviour of titanium in a corrosive ambient is known. This excelent resistance to corrosion is due to its capacity of forming a very fine film of protecting oxide on the surface thereof and a great speed for re-establishing the film if broken.

For forming a hip prosthesis, a series of conditions are necessary which have been previously defined as, suitable material for revising mechanical tensions to which it is subjected, to have suitable morphology, to have a fitting to the bone as solidly as possible, to be stable in order to avoid post-operatory luxations and finally that their adapting to each particualr case be possible.

Under three points of view the hip prosthesis may be described: Material, Design and Fitting.

The material, as seen hereinabove, the best indicated due to mechanical and physical properties and due to its resistance against corrosion is the alloy Ti-6Al-4V, which is preferably used for the object of the present invention, pertaining in turn to the group of materials called bio-materials, that is, to those which are compatible to the human organism, at the same time that the human organism is compatible to them.

The method for fitting the prosthesis to the bone will condition, logically, the morphology of the prosthesis. There are two systems for prosthetic fitting to the skeleton, with bone cement or without cement.

With the intention of improving still more the security of cemented prosthesis, the option is permitted of stems bathed in a fine film of PMMA which is polymerized together with bone cement which is used for its fitting.

A method for fitting the prosthesis consists of covering the surface of the prosthesis with a porous metal (in this case poropros) or the hydroxyapatite or poropatite (propros + hydroxyapatite) so that the osseous tissue, in the first case, penetrates within the pores and fixes the implant, and in the second, so that the joining osseous tissue is identified with prothesitic crystals.

In the zone covered by porous material of Titanium spheres (poropros) has a porosity of 20 or 30% and that in a future, when the bone has filling, the porous metal will force a 30% of the whole prosthetic surface, in this manner, in addition to the fitting "*per se*", there will exist a higher efficiency in the distribution of tensions and the sum of the spherular contacts gives rise to a more extended micro-mechanical union or prosthesis-skeleton.

On the other hand the use of stems with collar with a large inclination such as 40-50 implies higher quantity of cut bone.

In our case the angle of inclination of the collar is preferably 30º which is medially extended, allowing for a good access in cases of revision, in addition to preserving more the cortical bone.

The design of the present invention will be better understood with the aid of the following drawings:
Figure 1: Perspective view of a stem object of the present invention.
Figure 2: Elevation view of a stem with a section represnting a distant zone which shows the section of the latter with grooves and a plan view in which the elongated groove and the placing-taking off and orientation orifice, and a cross-section A'-A' in which the bed of the inlayed pore.

Its geometric shape offers an excellent stability in the reconstruction and restores a natural distribution of the charges, re-establishing the cinematics of the normal articulation.

Starting from the fact that the material of the stem is preferably of Ti-6Al-4V, forged and worked in cold, according to norms ASTM F-136 and F-620 which offers a high resistance to the fatigue and exceptional bio-compatibility.

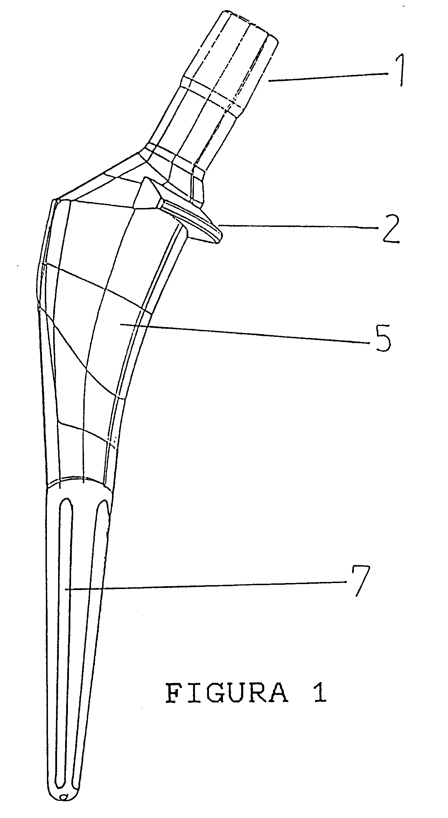

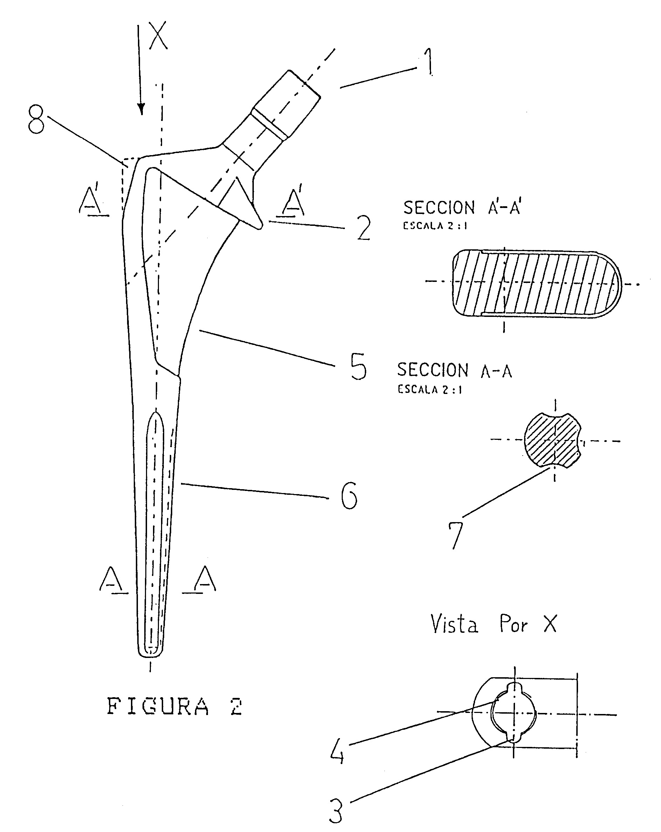

As it can be observed both in figure 1 and figure 2 this stem is composed of a neck zone at the upper part constituted by a European 12-14 morse (1) which permits accpeting femoral heads of various sizes and lengths, giving the same a modular character at international level since such a morse cone is normalized in many countris.

In the plan view of figure 2, it can be observed from a view from X, that it contains a groove (3), such that once the stem is located in the implant and it is rotated in order to occupy a more correct position, it is only sufficient to apply a key (impactor-orientor) the end of which being in the form of a shovel (such as the head of a flat screwdriver) into the grove, rotating the same until it is reaches the desired position.

On the other hand, the neck zone also has a calcareous support collar (2) which has a preferred inclination of 30º with respect to a horizontal plane which is medially extended, permitting a good access in the case of revision. This support, being less inclined, preserves more the cortical bone.

The cervical-diaphysary angle formed is preferably of 140º which is similar to the anatomical angle.

Inside said groove there is a threaded hole (4) (view from X in Fig. 2) used by means of an impactor-extractor, having one end provided with the same thread as that of hole (4) in order to take out the stem from its location in the body.

The middle zone of the stem is denominated proximity zone where a coating situated in the calcareous triangle (5) is inlayed, with the exception of the latteral zone, that is the coating is exclusively applied to the zones of osseous growth of the metaphysary zone of the femur (optimized metaphysary anchorage). This coating is preferably of polymetylmetacrylate (PMMA) or of spheres of titanium or the hydroxyapatite and more preferably of Poropatite (spheres of Titanium + Hydroxyapatite).

This zone has an angle in the lateral-proximal zone for an easy insertion ofthe stem with constant thickness sections.

The engagement of the stem is a forced adjustment of implant-bone, with an inclination-broadening in the lateral zone.

This broadening may present two ends at the rear proximal zone (8): a bevelled end and another of straight shape as seen in figure 2.

All this allows for optimum contact areas which give the result of a more uniform distribution of charges and a rigid fitting.

At the lower or distant zone a distant trunco-conical end (6) (tapering) is observed which eliminates the phenomenon of tension concentration is said distant part of the stem, thus permitting a more normal vascularization, facilitating the insertion of thesame in the femur and avoids pain at the distant zone which present other stems without such conicity.

It has a conical end with optimal angle preferably of 5º and a number of anti-rotational grovves (7) (fig. 2 section A-A) preferably 3 of them, being longitudinal and situated at 90º in front, rear and middle planes, and a rounded poit. All of which facilitating the positioning of the stem.

The surgical technic of the implantofthe stem is simple and precise using instrumentation corresponding to the hip modular system.

Having sufficiently described the nature of the present invention as well as one way of putting it into practice, it only remains to be added that it is possible to introduce changes of form, materials and disposition in the invention as a whole or in parts of which it is composed, as long as said alterations do not substantially vary the characteristics of the invention which are claimed as follows.

## Claims

1. Straight non-hardened stem for total hip prosthesis, characterized in being constituted by a neck zone with a European morse cone 12-14 which permits accepting femoral heads of various sizes and lengths, providing the same a modular character, a second proximal zone of forced adjustment of implant-bone with cross-sections of constant thickness and an inclination-broadening at a lateral zone. This broadening may present two ends at a rear proximal part thereof, bevelled at 15º or in straight shape; and a third distant zone of rounded end.

2. Staright non-hardened stem for total hip prosthesis, according to claim 1, characterized in that at the neck zone, the cervical-dyaphisary angle is similar to the anatomic angle, preferably between 135 and 145 degrees.

3. Staright non-hardened stem for total hip prosthesis, according to calim 1, characterized in that at the neck zone a support collar is medially extended having an angle of preferably between 25 and 35 degrees form a horizontal plane thus permitting a good access in the case of revision. This support of small inclination preserves more the cortical bone.

4. Straight non-hardened stem for total hip prosthesis, according to claim 1, characterized in that the neck zone has an elongated groove combined with a threaded orifice for taking out-implanting and orientation of the stem.

5. Straight non-hardened stem for total hip prosthesis, according to claim 1, characterized in that at the proximal zonethereof it has an inlayed coating preferably of poropatite (coating pore+hydroxypatite), another one which may be on the basis of titanium spheres only, of hydroxypatite or of polymethilmetalcrylate (PMMA), situated at the calcareous triangle with the exception of the latteral zone, which is optimum for stimulating osseous growth of the methaphysary zone of the femur.

6. Straight non-hardened stem for total hip prosthesis, according to claim 1, characterized in that its distant zone is tapered (trunco-conical) at an end thereof, having a high conicity, and grooved with anti-rotation grooves, which eliminate the concentration oftensions at said distant part of the stem and thus permits a more normal degree of vascularization, facilitating the insertion of same in the femur and avoids pain located at the distant zone which present other stems without said conocity.
